# EUROPÄISCHE PATENTSCHRIFT

(11) **EP 4 427 018 B1**
(45) Veröffentlichungstag und Bekanntmachung des Hinweises auf die Patenterteilung: **01.07.2026**
(21) Anmeldenummer: 22777657.2
(22) Anmeldetag: 26.09.2022
(51) Int. Cl.: G01N 1/32, B23K 31/12, G01N 1/34, G01N 21/84, G01N 1/28, G01N 21/71, G01N 1/44, G01N 25/72

(54) **VERFAHREN UND VORRICHTUNG ZUR SCHLIFFBILDPRÄPARATION EINER METALLURGISCHEN PROBE**
METHOD AND DEVICE FOR PREPARING METAL SPECIMENS FOR PRODUCING MICROGRAPHS
MÉTHODE ET DISPOSITIF DE PRÉPARATION D'UN ECHANTILLON DE MÉTAL POUR MICROGRAPHIE

(30) Priorität: 03.11.2021 DE 102021212385
(43) Veröffentlichungstag der Anmeldung: 11.09.2024
(73) Patentinhaber: Robert Bosch GmbH, 70442 Stuttgart (DE)
(72) Erfinder: BORMANN, Axel, 96049 Bamberg (DE); STRAUBMEIER, Christoph, 96052 Bamberg (DE); QUEST, Dennis, 96050 Bamberg (DE); AROLD, Markus, 96158 Frensdorf (DE)
(86) Internationale Anmeldenummer: PCT/EP2022/076676
(87) Internationale Veröffentlichungsnummer: WO 2023/078614

(56) Entgegenhaltungen:
- WO-A1-2013/117667
- US-A1- 2013 081 882

## Beschreibung

Die vorliegende Erfindung betrifft ein Verfahren zur Schliffbildpräparation einer metallurgischen Probe, wobei nach Aufbereitung der Probe eine thermische Kontrastierung der Oberfläche durch Lasermittel vollzogen wird. Außerdem betrifft die Erfindung auch eine Vorrichtung zur Durchführung dieses Verfahrens zur Schliffbildpräparation.

Das Einsatzgebiet der Erfindung erstreckt sich vornehmlich auf die Qualitätsprüfung von metallischen Werkstücken, insbesondere im Bereich bauteilverbindender Schweißnähte. Für die Beurteilung von Metall-Schweißverbindungen werden üblicherweise die hier interessierenden metallurgischen Schliffbilder angefertigt, welche beispielsweise mithilfe eines Lichtmikroskops in an sich bekannter Weise materialografisch analysierbar sind. Im Schliffbild werden Schweißnähte, Gefüge- und Korngrenzen, Wärmeeinflusszonen, Einschweißtiefen, Anbindebreiten, Fügespalte und dergleichen sichtbar.

### Stand der Technik

Gemäß dem allgemein bekannten Stand der Technik werden solche Schliffbilder bislang durch Einbetten einer Probe des Werkstücks in Kunststoff, Auftrennen der Probe und Durchführung mehrerer Schleifprozesse bis hin zum Polieren und Ätzen präpariert, um ein aussagekräftiges Schliffbild zu erhalten.

Dies erfolgt unter Zuhilfenahme verschiedener Werkzeuge in einem recht zeitaufwendigen Prozess. Häufig kann nicht die gewünschte Qualität eines Schliffbildes erzielt werden, die eine eindeutige Erkennung sowie Aussage über die vorgenannten Kenngrößen erlaubt. So können undifferenzierte dunkle Flächen im Schliffbild entstehen, welche keine Unterscheidung zwischen Schweißnaht, Wärmeeinflusszonen und dem Grundgefüge des Werkstücks zulassen. Andererseits können zu starke Kontrastunterschiede im Schliffbild die Auswertung ebenfalls erschweren. Zudem kommen für den letzten Präparationsschritt eines Ätzens gewöhnlich chemische Ätzmittel zum Einsatz, welche einer besonderen Behandlung bedürfen und gesundheitsschädlich sind. Durch die für die herkömmliche Schliffbildpräparation zum Einsatz kommenden spanenden Bearbeitungsschritte der Oberfläche können schmale Fügespalte durch abgetragenes Material zugesetzt werden und sind daher nicht mehr erkennbar.

Aus der WO 2013/117667 A1 geht ein gattungsgemäßes Verfahren zur Schliffbildpräparation hervor, welches anstelle eines chemischen Ätzens auf einem Laserätzen basiert. Hierbei wird die Oberfläche einer materialografisch zu analysierenden metallurgischen Probe partiell einem Poliervorgang unterzogen und anschließend mit mindestens einem Laserstrahl derart bearbeitet, dass aufgrund der thermischen Ausdehnung der Körner der bearbeiteten Probenoberfläche ein Gefügebild erhalten wird, welches materialografisch analysierbar ist. Die Bearbeitung der Probenoberfläche erfolgt vorzugsweise mit mindestens einem Laserstrahl unter Schutzgasatmosphäre. Im Rahmen des Laserätzens wird die Probenoberfläche partial rasterartig bearbeitet. Mithilfe des Laserstrahls eines Faserlasers, lasergeführten Diodenlasers oder Kohlendioxid-Lasers wird auf die Probenoberfläche eine bestimmte Energiedichte aufgebracht, wobei sich abhängig vom Wärmeausdehnungskoeffizienten des Probenmaterials die Körner der bearbeiteten Oberfläche ausdehnen und an den Korngrenzen zugleich ein Abdampfen aufgrund der größeren Fehlstellendichte im Kristallgitter ergibt, also ein Materialabtrag stattfindet. Die Bearbeitung der Probenoberfläche erfolgt hierbei vorzugsweise unter atmosphärischen Bedingungen oder Sauerstoff-Zugabe. Das Gefüge wird dadurch sichtbar verstärkt, wobei die Kontrastverstärkung bis zur Oxidationsgrenze erfolgt.

Dennoch findet im Rahmen dieser nicht-chemischen Schliffbildpräparation eine dem Laserätzen vorausgehende mechanische Bearbeitung der Oberfläche durch Polieren statt, wozu verschiedene Schleif- und Poliergeräte mit abnehmender Körnung zum Einsatz kommen. Außerdem wird im Rahmen der Schliffbildpräparation eine Reinigung der polierten Oberfläche durch ein Ultraschallbad vorgeschlagen. Insgesamt kommen also eine Reihe von Bearbeitungsmaschinen und Werkzeugen zum Einsatz, woraus eine gerätetechnisch aufwendige und zeitintensive Schliffbildpräparation von metallurgischen Proben resultiert.

Es ist daher die Aufgabe der vorliegenden Erfindung, ein Verfahren sowie eine Vorrichtung zur Schliffbildpräparation einer metallurgischen Probe zu schaffen, welches/welche eine fertigungstechnisch einfache und schnelle Schliffbildpräparation gewährleistet.

### Offenbarung der Erfindung

Die Aufgabe wird verfahrenstechnisch durch Anspruch 1 gelöst. Der nebengeordnete Vorrichtungsanspruch 6 gibt eine geeignete Apparatur zur Durchführung des erfindungsgemäß mehrschrittigen Verfahrens an. Die jeweils rückbezogenen abhängigen Ansprüche widmen sich vorteilhaften Weiterbildungen der Erfindung.

Die Erfindung schließt die verfahrenstechnische Lehre ein, dass zur Schliffbildpräparation einer metallurgischen Probe, bei welcher nach Aufbereitung der Probe eine thermische Kontrastierung der Oberfläche durch Lasermittel anstelle chemischer Ätzmittel vollzogen wird, die gesamte Schliffbildpräparation inklusive Aufbereitung mit einem Ultrakurzpuls(UKP)-Laser durchgeführt wird, welcher zumindest die nachfolgenden Bearbeitungsschritte durchführt:
- Reinigen R der Oberfläche der Probe mit einer Abtragtiefe zwischen 1 bis 50 µm zum Freilegen von Fügespaltstrukturen,
- Kontrastieren K der gereinigten Oberfläche der Probe mit einer Abtragtiefe zwischen 0,5 bis 20 µm zur Sichtbarmachung von Gefügestrukturen,
- Laserätzen A der kontrastierten Oberfläche der Probe durch abtragloses Erzeugen thermischer Anlauffarbstruktur und/oder oberflächlicher Oxidation.

Dabei entsteht eine thermische Anlauffarbstruktur durch Erhitzen der Probe, bis auf der Oberfläche Anlauffarben sichtbar werden. Solche Anlauffarben, auch Anlassfarben genannt, sind oberflächliche, irisierende bunte Färbungen eines Stoffes, die durch Interferenz an dünnen Schichten entstehen. Sie finden sich hauptsächlich bei Metallen, aber auch auf Mineralien. Diese Interferenz ist denen in Ölflecken auf Pfützen oder in Lamellen von Seifenblasen sehr ähnlich, und lässt Rückschlüsse auf die Materialeigenschaften zu.

Unter einer Gefügestruktur wird der Verband der Kristallite und Körner in einem Metall verstanden. Da die Kristallisation häufig an vielen Punkten der Schmelze gleichzeitig beginnt, besteht ein Stück Metall aus einer Vielzahl einzelner Kristalle, die aneinandergewachsen sind. Gefügemerkmale sind beispielsweise die Korngröße, die Kornform sowie unterschiedliche Phasen oder Ausscheidungen. Die Ausbildung des Gefüges wird durch viele Faktoren wie beispielsweise durch den Gehalt an Legierungselementen oder die Herstellungs- oder Verarbeitungsbedingungen bestimmt. Hieraus lassen sich entsprechende Materialeigenschaften ablesen und prüfen.

Unter Fügespaltstruktur werden Anordnungen von Fügespalten verstanden, die zwischen benachbart aneinander liegenden Bauteilen entstehen. Ein Fügespalt kann dabei von einer bauteilverbindenden Schweißnaht unterbrochen sein.

Der Vorteil der erfindungsgemäßen Lösung liegt insbesondere darin, dass nicht nur das chemische Ätzen durch ein Laserätzen ersetzt wird, sondern auch die vorausgehende Probenaufbereitung lasertechnisch erfolgt, was im Rahmen der Erfindung ein einziger UKP-Laser möglich macht, der für die verschiedenen Bearbeitungsschritte lediglich mit entsprechend angepassten Parametern betrieben wird.

Ist die Oberfläche der metallurgischen Probe beispielsweise sägerau, so kann dem Reinigen R optional ein Glätten G der Oberfläche vorangestellt werden, um diese hinreichend einzuebnen, also die Sägeriefen zu entfernen. Das optionale Glätten G ebnet also Unebenheiten ein, wie diese bei sägerauen Schnitten entstehen können und bietet eine Basis für den nachfolgenden Aufbereitungsprozess. Herkömmliche kaskadische Schleifschritte sowie ein finales Polieren können hierdurch entfallen. Die Wahl der Parameter für den UKP-Laser erfolgt für das Glätten G derart, dass Schneidriefen, Unebenheiten und dergleichen vollständig entfernt werden, was vorzugsweise durch Mehrfachüberfahren mit dem Laserstrahl bei einer Abtragtiefe zwischen 1 bis 150 µm, ganz vorzugsweise 20 bis 150 µm, durchgeführt wird, je nach dem in welchem Vorbearbeitungszustand die Probe sich befindet. Um eine raue Oberfläche durch Glätten einzuebnen, bedarf es eines hinreichenden Materialabtrags. Ist die Probe schon vorgeschliffen und poliert, so genügt in der Regel eine einzige Überfahrt mit dem UKP-Laser, um die Oberfläche hinreichend einzuglätten. Da dies nicht vollständig abtragsfrei geschehen kann, gilt für das Glätten eine Untergrenze von 1µm Abtragtiefe. Wird nach 150 µm keine homogene eingeglättete Oberfläche erzielt muss die Probe gegebenenfalls mechanisch vorbehandelt werden, beispielsweise durch Schleifen.

Das nachfolgende Reinigen R bietet die Möglichkeit, durch die Vorbehandlung zugesetzte Fügespalte freizulegen. Für den Verfahrensschritt des Reinigens R erfolgt die Wahl der Laserparameter so, dass der Materialabtrag gerade eben die Fügespaltstrukturen sichtbar freilegt, welche durch die vorherige Bearbeitung zugesetzt sein könnten. Hierzu erfolgt ebenfalls ein Mehrfachüberfahren mit dem Laserstrahl mit einer Abtragtiefe zwischen 1 bis 50 µm. Um Rückstände aus Fügespalten zu entfernen, muss der Abtragsprozess mit einer gewissen Leistungsdichte gefahren werden. Hierdurch geht auch immer etwas Material an der Probe selbst verloren. Mehr als 50 µm Abtragtiefe machen als Bereichsobergrenze deshalb keinen Sinn, da die Probe in der Regel bereits eben und vorbehandelt ist. Für das Reinigen wird in Abgrenzung zum Glätten bei derselben Probe eine demgegenüber größere Abtragtiefe gewählt.

Das nachfolgende Kontrastieren K erlaubt zusätzlich eine Sichtbarmachung von beispielsweise Schweißnahtdurchmischungen, Kontrastreduzierungen der Wärmeeinflusszone, diffuser Kontrastierung in Form von Mattierungen und dergleichen. Hierdurch wird die Sichtbarkeit von Schweißnähten weiter verbessert. Dieses spezielle Kontrastieren K erfolgt mit Laserparametern, die derart eingestellt sind, dass eine homogene matte Oberfläche erzeugt wird, was durch Ein- oder Mehrfachüberfahren mit dem Laserstrahl mit einer Abtragtiefe zwischen 0,5 bis 20 µm erzielt wird. Für das Kontrastieren ist zumeist nur ein einmaliges zeilenweise Überfahren der Oberfläche mit dem UKP-Laser nötig, mit welchem der angegebene untere Grenzwert der Abtragtiefe von 0,5 µm realisierbar ist. Bei einem n-fachen Überfahren in vorzugsweise unterschiedlichen Schraffurrichtungen erhöht sich die Abtragtiefe mit dem Faktor n. In geringer Weise ist diese alternativ oder zusätzlich natürlich auch über die Laserleistung, die Fluenz, variierbar. Wie beim Glätten und Reinigen dient der obere Grenzwert als Abgrenzungsmerkmal zu den anderen Verfahrensschritten.

Das finale Laserätzen A erlaubt eine thermische Probenpräparation ohne flüssige Ätzmittel mittels Oxidation von Verunreinigungen an Korn-/Schmelz-Grenzen zur Sichtbarmachung feinerer Oberflächenstrukturen. Dies erfolgt unter einer Luft- oder Sauerstoffatmosphäre als Prozessgas, wobei der Laserstrahl defokussiert eingestellt ist und der Vorschub vorzugsweise zeilenförmig in mäanderförmiger Weise durchgeführt wird. Hierbei findet kein Materialabtrag statt. Es wird vielmehr gezielt Wärme in die Oberfläche der metallurgischen Probe eingebracht und mittels Prozessgas die Oberfläche kontrolliert oxidiert. Da Verunreinigungen an Korngrenzen, Schmelzzonenübergängen und dergleichen oberflächlich chemisch stärker reagieren sowie ein Mikrowärmestau entsteht, wird das Gefüge bzw. die Schweißnaht sichtbar.

Die erfindungsgemäße Lösung erlaubt zum Laserätzen die Verwendung desselben Laserwerkzeugs, wie dieses auch zur vorausgegangenen Probenaufbereitung genutzt worden ist, was eine erhebliche verfahrenstechnische Vereinfachung darstellt und durch Wegfall von Werkzeugwechseln eine schnelle Schliffbildpräparation der metallurgischen Probe ermöglicht. Daneben besteht der Vorteil, dass die erfindungsgemäße Lösung auch geeignet ist zur Anwendung an bereits vorhandenen Schliffen, auch nur polierten, geschliffenen oder auch nur getrennten Probenoberflächen. So ist das optionale Glätten G in der Regel nur zur Beseitigung von rauen Oberflächen vorzusehen, was eine flexible Anwendung je nach Probenbeschaffenheit gewährleistet. Der herkömmliche Präparationsprozess durch Schleifen, Polieren und Ätzen wird hierdurch ersetzt oder zumindest deutlich vereinfacht. Denn im Prinzip kann die erfindungsgemäße Lösung je nach Bedarf in unterschiedlichen Stadien der Schliffbildpräparation - von sägerau bis hin zu fertig kontrastiert - angewendet werden, um ein aussagekräftiges metallurgisches Schliffbild zu schaffen.

Die Parameter für die einzelnen Bearbeitungsschritte sind typischer Weise für jede Schweißverbindung aufgrund unterschiedlicher Gegebenheiten, wie Materialien der Fügepartner, Wärmeleitfähigkeit, Größe, Vorbehandlungszustand, individuell zu entwickeln und zu erproben. Prinzipiell wird eine minimale bis maximale Anzahl an Überfahrten durchgeführt, bis für jeden Bearbeitungsschritt das Stoppkriterium einer hinreichend gut geglätteten, gereinigten beziehungsweise kontrastierten Oberfläche erreicht ist. Dies bemisst sich anhand subjektiver Inaugenscheinnahme der Probenoberfläche nach jedem Bearbeitungsschritt.

Für das Glätten gilt als Stoppkriterium eine riefenfreie saubere Oberfläche. Der Bereich der Schweißnaht und angrenzende Bereiche, welche für die Bewertung von Einschweißtiefe oder Anbindebreite relevant sind, sind eben und vorzugsweise vorbehandlungsfrei, das heißt Spuren einer Vorbehandlung sind nicht mehr sichtbar. Dies lässt sich anhand einer hilfsmittellosen Sichtprüfung beurteilen.

Für das Reinigen erfolgt die Prüfung unter dem Mikroskop. Stoppkriterium ist beispielsweise die Freilegung des Fügespalts. Dabei ist der Fügespalt deutlich sichtbar und Grate oder Schleifreste sind vollständig entfernt.

Für das Kontrastieren wird das Stoppkriterium geprüft, ob eine Sichtbarkeit der Schweißnaht oder ein hinreichender Kontrast zum Grundgefüge zur eindeutigen Lagebestimmung der Schweißnaht vorhanden ist.

Die Erfolgskontrolle erfolgt demnach in der Regel manuell optisch und außer für das Glätten mit Hilfe eines Auflicht-Mikroskops. Daneben ist auch eine automatische Beurteilung per Mustervergleich oder dergleichen denkbar.

Vorzugsweise wird der UKP-Laser zum Laserätzen A mit einer konstanten Wellenlänge Λ im Bereich zwischen 1020 Nanometern (nm) bis 1040 nm, vorzugsweise 1030 nm mit einer Toleranz von gewöhnlich +/- 3 nm, betrieben, kombiniert mit einer Impulsdauer t_{P} von ≤ 10 Pikosekunden (ps). Im Gegensatz zum Stand der Technik, worin zum Laserätzen ein cw- oder ns-gepulster Laser beschrieben ist, können mit dem im Rahmen der vorliegenden Erfindung eingesetzten UKP-Laser die vorgenannt spezifizierten Betriebsparameter erzielt werden.

Gemäß einer weiteren die Erfindung verbessernden Maßnahme wird vorgeschlagen, dass zumindest die materialabtragenden Schritte Reinigen R und Kontrastieren K nach Maßgabe eines gleichförmig linearen Abtragmusters durchgeführt werden. Dieses Abtragmuster lässt sich durch das Scansystem der Vorrichtung erzeugen. Hierbei wird das Muster im Winkel von vorzugsweise 45° mit mindestens sechs Schraffurrichtungen, vorzugsweise acht Schraffurrichtungen, pro Abtrag aufgebracht. Dabei werden je nach Anforderung die Betriebsparameter Anzahl an Wiederholungen, Schraffurabstand, Laserleistung, Fokuslage, Scangeschwindigkeit, Schutzgas u.a. variiert, um für das Reinigen R und Kontrastieren K sowie auch für das optionale Glätten G einen definiert tiefen, homogenen Abtrag zu erhalten.

Das vorstehend beschriebene mehrschrittige Verfahren zur Schliffbildpräparation mit einem UKP-Laser lässt sich mit einer Vorrichtung umsetzen, die neben dem UKP-Laser zur Erzeugung eines Laserstrahls auch eine Strahlablenkeinheit, ein sogenannter Scan-Kopf, zur Bewegung und Fokussierung des Laserstrahls entlang der Oberfläche der metallurgischen Probe, eine Probeneinspanneinheit zur Positionierung der metallurgischen Probe relativ zur Strahlablenkeinheit sowie eine elektronische Steuereinheit zur koordinierten Ansteuerung zumindest der vorgenannten Einheiten der Vorrichtung nach Maßgabe des durchzuführenden Verfahrensschritts G; R; K; A umfasst.

Die koordinierte Ansteuerung der Einheiten erfolgt derart, dass stets im defokussierten Bereich gearbeitet wird. Die Schritte G; R; K unterscheiden sich dahingehend, dass beim Glätten mehr Überfahrten als beim Reinigen, als beim Kontrastieren benötigt werden. Unter einem koordinierten Ansteuern wird hier die Programmierung der Abtragsgeometrie verstanden, welche durch Parameter, wie Form, Füllmuster der Form (Abtragmuster in unterschiedlichen Schraffurrichtungen), Leistung, Bearbeitungsgeschwindigkeit, Sprunggeschwindigkeit, Schraffurwinkel, Wiederholungen, Defokussierung, Sprung-/ Start- und Stopp-Eigenschaften (beispielsweise LaserOnDelay, LaserOffDelay, Skywriting) definiert ist.

Die Fluenz sollte beim Glätten 0,6 J/cm² mit einer Toleranz von +/-0,1 J/cm² betragen, um beispielsweise mit etwa 6 Watt Laserleistung ein gutes Bearbeitungsergebnis zu erhalten, also Materialaufschäumung oder Kegelbildung aufgrund Fehlstellen zu vermeiden. Für das Kontrastieren und Reinigen kann hiervon abgewichen werden und von vergleichsweise weniger Laserleistung ausgegangen werden, beispielsweise reichen hierfür je nach Apparatur bereits 4 bis 5 Watt aus, was zu einer entsprechend verringerten Fluenz führt.

**Speziell** beim Ätzen wird im Vergleich zu den vorgenannten Verfahrensschritten noch stärker defokussiert, um einen größeren Spotdurchmesser auf der Bauteiloberfläche zu erhalten. Es soll hierdurch eine lokale Erwärmung bis zum üblichen materialabhängigen Anlasstemperaturbereich erzeugt werden, um die Korngrenzen mittels Anlassfarben optisch sichtbar zu machen. Der Laser wirkt hierbei somit nicht mehr abtragend, sondern nur noch thermisch.

Die koordinierte Ansteuerung der verschiedenen Einheiten der Vorrichtung beinhaltet eine Platzierung der Probe in der Probeneinspanneinheit sowie ein Verfahren der Achsen der Probeneinspanneinheit, so dass die Fokusposition des Laserstrahls orientiert zur Bauteiloberfläche liegt, vorzugsweise defokussiert -1,7 mm unterhalb Bauteiloberfläche für die Probenaufbereitung im Rahmen der Schritte G, R, K. Mit der Strahlablenkeinheit wird nach Maßgabe eines Scanprogramms eine definierte Auslenkung des Laserstrahls zur Mustererzeugung durchgeführt.

Zum Beispiel wird für ein Bearbeiten eines Quadrates von 4x4mm Kantenlänge auf der Probenoberfläche mit dem UPK-Laser eine Laserleistung von 6 W bei 400 kHz Pulsfolgefrequenz erzeugt. Das Abfahren der Pfade des Abtragmusters erfolgt mit einem Vorschub von 2 m/s. Dabei beträgt der Linienabstand des Abtragmusters 0,02 mm. Zunächst wird nur in eine Richtung bearbeitet. Bei einer Richtungsanzahl von 8 ergibt sich ein sogenannter Hash-Winkel von 45°. Mit anderen Worten wird das erzeugte Linienmuster in Summe 8 mal ausgeführt und dabei zwischen jedem Schritt um 45° gedreht, um einen homogenen Abtrag ohne Vorzugsrichtung zu erzielen.

Diese 8 Schritte pro Schraffurebene werden so oft wiederholt, bis die gemäß Scanprogramm vorgegebene Abtragtiefe erzielt ist. Anschließend wird das Scanprogramm gestoppt und die Probe zur Entnahmeposition gefahren, um das Ergebnis des Bearbeitungsschritts manuell optisch zu prüfen. Ist das Ergebnis noch nicht befriedigend, erfolget eine Fortsetzung der Bearbeitung.

Zusätzlich kann in der Vorrichtung zwischen dem UKP-Laser und der Strahlablenkeinheit ein sogenannter Fokusshifter, beispielsweise eine Varioscaneinheit (varioSCAN ^{®} der Firma Scanlab) angeordnet werden. Der Fokusshifter beinhaltet eine Fokussieroptik, welche eine hochdynamische und äußerst präzise Positionierung des Laserfokus entlang der optischen Z-Achse ermöglicht. Hierdurch lassen sich XY-Lasereinheiten in einfacher Weise zu 3D-Strahlablenksystemen erweitern. Der Laserfokus kann dabei entlang der Kontur des zu bearbeitenden Werkstücks geführt werden. Hierfür ist die Fokussieroptik motorisch verfahrbar, um zusätzlich Arbeitsabstand sowie Spotgröße zum Defokussieren stufenlos einzustellen.

Ferner kann außerdem zwischen dem UKP-Laser und dem Fokusshifter eine Strahlaufweiteeinheit zum Vergrößern oder Verkleinern des Laserstrahlquerschnitts angeordnet werden. Hiermit wird ein kollimierter Laserstrahl aufgenommen und in seinem Querschnitt vergrößert beziehungsweise verkleinert.

Gemäß einer weiteren, die Erfindung verbessernden Maßnahme wird vorgeschlagen, dass die Probeneinspanneinheit mit einer CNC-Kinematik ausgestattet ist, um die zu bearbeitende Probe räumlich gegenüber der relativ ortsfest hierzu angeordneten Strahlablenkeinheit zu positionieren. Hierdurch lässt sich sicherstellen, dass die Probenoberseite im Fokus des Laserstrahls liegt. Daneben ist es jedoch auch denkbar, dass eine manuelle Einrichtung durchgeführt wird.

Die Abtragtiefe wird prinzipiell über die Anzahl der Überfahrten variiert, wobei sich die Abtragstiefe pro Überfahrt als eine Funktion aus Laserleistung und Strahldurchmesser am Bearbeitungsort, Scan-Geschwindigkeit, Material, Linienabstand des Hash-Musters, Winkel der Hash-Muster und Anzahl der Hash-Muster beschreiben lässt. Die resultierende Abtragstiefe von Zehntel-Mikrometern bis wenigen Mikrometern ist dann als Prozessergebnis über den abgescannten Bereich gesehen im Wesentlichen konstant.

### Kurzbeschreibung der Zeichnung

Weitere die Erfindung verbessernde Maßnahmen werden nachstehend gemeinsam mit der Beschreibung eines bevorzugten Ausführungsbeispiels der Erfindung anhand der Figuren näher dargestellt. Es zeigt:
- Fig. 1: eine schematische Darstellung einer Vorrichtung zur Durchführung einer Schliffbildpräparation einer metallurgischen Probe per UKP-Laser,
- Fig. 2: einen Ablaufplan von Verfahrensschritten G; R; K; A, welche mit der Vorrichtung gemäß Fig. 1 durchgeführt werden, und
- Fig. 3: ein Schliffbild einer metallurgischen Probe in Ergebnis der erfindungsgemäßen Bearbeitungsschritte.

### Detailbeschreibung der Zeichnung

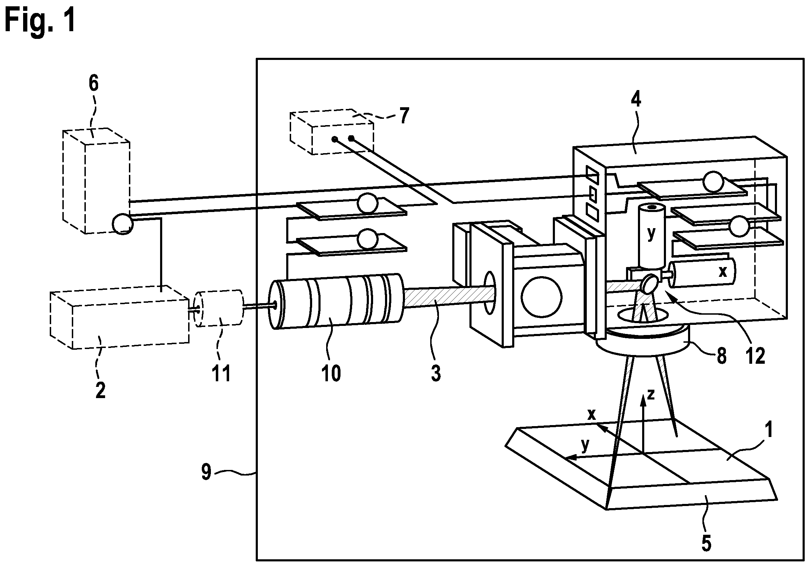

Gemäß Fig. 1 besteht eine Vorrichtung zur Schliffbildpräparation einer metallurgischen Probe 1 im Wesentlichen aus einem UKP-Laser 2, der mit einer Impulsdauer t_{P} von ≤ 10 ps bei einer Wellenlänge Λ von 1030 nm betrieben wird und der Erzeugung eines Laserstrahls 3 dient, welcher über eine Strahlablenkeinheit 4 zur Bewegung und Fokussierung des Laserstrahls 3 relativ zur Oberfläche der Probe 1 geleitet wird. Die Probe 1 ist mittels einer Probeneinspanneinheit 5 relativ zur Strahlablenkeinheit 4 positioniert und mit einer - hier nicht weiter dargestellten - CNC-Kinematik ausgestattet, um die zu bearbeitende Probe 1 räumlich entsprechend der dargestellten Achsen x, y, z gegenüber der relativ ortsfest hierzu angeordneten Strahlablenkeinheit 4 zu positionieren, welche insoweit nach Art eines Laserkopfes ausgeführt ist.

Zur koordinierten Ansteuerung der Komponenten oder Einheiten der Vorrichtung dient eine elektronische Steuereinheit 6, die mit den Einheiten oder Komponenten des Systems in kommunikativer Verbindung steht. Die Stromversorgung erfolgt über eine Stromversorgungseinheit 7. Die Vorrichtung bildet ein 2(3)D-Galvano-Scansystem, das mit einem f-Theta-Objektiv 8 zum Fokussieren des Laserstrahls 3 ausgestattet ist. Das mit der Vorrichtung auszuführende mitunter mehrschrittige erfindungsgemäße Verfahren stellt einen Laserklasse-4-Prozess dar, welcher innerhalb eines geschlossenen Gehäuses 9 untergebracht und damit laserdicht gekapselt ist. Außerhalb des geschlossenen Gehäuses 9 herrscht Laserklasse 1.

Weiterhin ist zwischen dem UKP-Laser 2 und der Strahlablenkeinheit 4 ein Fokusshifter 10 zum Positionieren des Laserfokus entlang der optischen Z-Achse des Laserstrahls 3 angeordnet. Zwischen dem UKP-Laser 2 und dem Fokusshifter 10 befindet sich eine zusätzliche Strahlaufweiteeinheit 11 zum Vergrößern oder Verkleinern des Laserstrahlquerschnitts, ehe dieser in den Bereich des geschlossenen Gehäuses 9 eintritt.

Die Strahlablenkeinheit 4 weist ein zweiachsiges Spiegelsystem 12 zum Steuern des Laserstrahls 3 entlang der zu bearbeitenden Oberfläche der Probe 1 gemäß eines durch die elektronische Steuereinheit 6 vorgegebenen Abtragmusters auf.

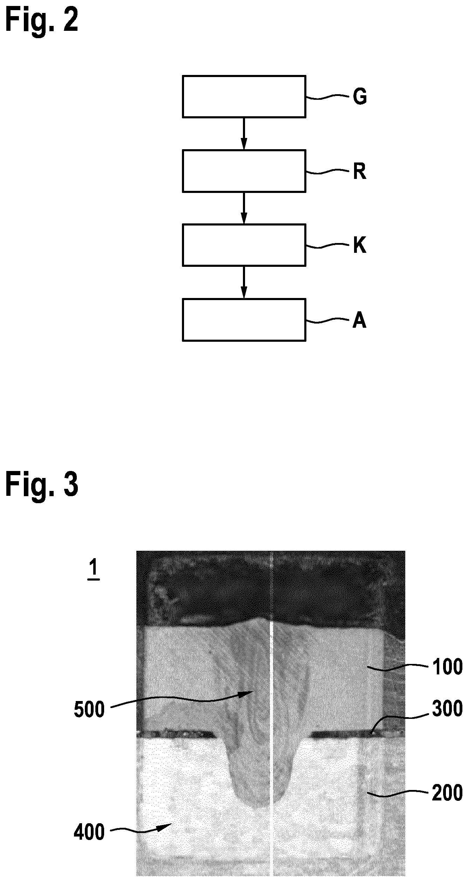

Gemäß Fig. 2 lässt sich mit der vorstehend beschriebenen Vorrichtung eine Schliffbildpräparation der metallurgischen Probe 1 durchführen, welche bei diesem Ausführungsbeispiel von einer Probe 1 mit Schneidriefen, also einer sehr rauen Oberfläche, ausgeht. Es erfolgt in einem ersten Schritt ein Glätten G der Oberfläche der Probe 1 zum Einebnen der Oberfläche, so dass die Schneidriefen durch Mehrfachüberfahren der Oberfläche bei einer Abtragtiefe von 20 bis 150 µm vollständig entfernt sind.

Nach diesem Einebnen erfolgt in einem zweiten Schritt ein Reinigen R der Oberfläche der Probe 1, welches zum Freilegen von Fügespaltstrukturen an der Oberfläche der Probe 1 dient. Hierzu erfolgt ebenfalls ein Mehrfachüberfahren mit dem Laserstrahl 3 bei einer Abtragtiefe zwischen 1 bis 50 µm, bis eventuell vorhandene Fügespaltstrukturen sichtbar werden.

In einem dritten Schritt wird ein Kontrastieren K der gereinigten Oberfläche der Probe 1 zur Sichtbarmachung von Gefügestrukturen durchgeführt. Dies erfolgt ebenfalls durch Mehrfachüberfahren mit dem Laserstrahl 3 bei einer Abtragtiefe zwischen 0,5 bis 20 µm. Es wird hierdurch eine ebene, homogen matte Oberfläche erzeugt.

In einem vierten und letzten Schritt wird ein Laserätzen A der kontrastierten Oberfläche der Probe 1 durch den Laserstrahl 3 unter Luft oder Sauerstoff als Prozessgas durchgeführt, wobei der Laserstrahl defokussiert wird und zeilenförmig in mäanderförmiger Weise über die Oberfläche der Probe 1 geführt wird. Hierdurch wird gezielt Wärme in die Probe 1 eingebracht und mittels Prozessgas die Oberfläche kontrolliert oxidiert, um das Gefüge oder eine enthaltene Schweißnaht sichtbar zu machen. Anschließend erfolgt in an sich bekannter Weise eine materialografische Auswertung des präparierten Schliffbildes, beispielsweise mit einem Lichtmikroskop.

Die Fig. 3 zeigt ein exemplarisches Schliffbild, in welchem miteinander verschweißte Bauteile 100 und 200 und der dazwischenliegende Fügespalt 300 sichtbar ist, welcher infolge des Reinigens R kontraststark hervortritt. Außerdem macht das Kontrastieren K Gefügestrukturen 400 an der Probenoberfläche sichtbar und das Laserätzen A führt dazu, dass thermische Anlauffarben 500 im Schweißnahtbereich optisch hervortreten.

Die Erfindung ist nicht beschränkt auf das vorstehend beschriebene bevorzugte Ausführungsbeispiel. Es sind vielmehr auch Abwandlungen hiervon denkbar, welche vom Schutzbereich der nachfolgenden Ansprüche mit umfasst sind. So ist es beispielsweise auch möglich, dass das Glätten G der Oberfläche einer Probe entfallen kann, falls die Probe in der Ausgangssituation bereits hinreichend eben ist.

## Patentansprüche

1. Verfahren zur Schliffbildpräparation einer metallurgischen Probe (1), bei welcher nach Aufbereitung der Probe (1) eine thermische Kontrastierung der Oberfläche durch Lasermittel vollzogen wird,
**dadurch gekennzeichnet, dass**
durch einen UKP-Laser (2) zumindest die nachfolgenden Bearbeitungsschritte durchgeführt werden:
- Reinigen (R) der Oberfläche der Probe (1) mit einer Abtragtiefe zwischen 1 bis 50 µm zum Freilegen von Fügespaltstrukturen (300),
- Kontrastieren (K) der gereinigten Oberfläche der Probe (1) mit einer Abtragtiefe zwischen 0,5 bis 20 µm zur Sichtbarmachung von Gefügestrukturen (400),
- Laserätzen (A) der kontrastierten Oberfläche der Probe (1) durch abtragloses Erzeugen thermischer Anlauffarbstruktur (500) und/oder oberflächlicher Oxidation.

2. Verfahren nach Anspruch 1,
**dadurch gekennzeichnet, dass**
vor dem Reinigen (R) ein Glätten (G) der Oberfläche der Probe (1) mit einer Abtragtiefe zwischen 20 bis 150 µm zum Einebnen rauer Oberflächen durchgeführt wird.

3. Verfahren nach Anspruch 1,
**dadurch gekennzeichnet, dass**
der UKP-Laser (2) zum Laserätzen (A) mit einer konstanten Wellenlänge 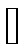 im Bereich zwischen 1020 nm bis 1040 nm, vorzugsweise 1030 nm +/- 3 nm, sowie mit einer Impulsdauer (t_{P}) von ≤ 10 ps betrieben wird.

4. Verfahren nach Anspruch 1 oder 2,
**dadurch gekennzeichnet, dass**
zumindest die materialabtragenden Schritte Reinigen (R) und Kontrastieren (K) nach Maßgabe eines gleichförmig linearen Abtragmusters durchgeführt werden.

5. Verfahren nach Anspruch 4,
**dadurch gekennzeichnet, dass**
das Abtragmuster mit mindestens 6 Schraffurrichtungen, vorzugsweise 8 Schraffurrichtungen, im Winkel von 40° bis 50°, vorzugsweise 45°, pro Abtrag aufgebracht wird.

6. Vorrichtung zur Durchführung des mehrschrittigen Verfahrens nach einem der vorstehenden Ansprüche, zumindest umfassend einen UPK-Laser (2) zur Erzeugung eines Laserstrahls (3), eine Strahlablenkeinheit (4) zur Bewegung und Fokussierung des Laserstrahls (3) relativ zur Oberfläche der Probe (1), eine Probeneinspanneinheit (5) zur Positionierung der Probe (1) relativ zur Strahlablenkeinheit (4), eine elektronische Steuereinheit (6) zur koordinierten Ansteuerung dieser Einheiten nach Maßgabe des durchzuführenden Verfahrensschritts (G; R; K; A).

7. Vorrichtung nach Anspruch 6,
**dadurch gekennzeichnet, dass** zwischen dem UPK-Laser (2) und der Strahlablenkeinheit (4) ein Fokusshifter (10) zum Positionieren des Laserfokus entlang der optischen Z-Achse angeordnet ist.

8. Vorrichtung nach Anspruch 7,
**dadurch gekennzeichnet, dass** zwischen dem UPK-Laser (2) und dem Fokusshifter (10) eine Strahlaufweiteeinheit (11) zum Vergrößern des Laserstrahlquerschnitts angeordnet ist.

9. Vorrichtung nach Anspruch 6,
**dadurch gekennzeichnet, dass** die Strahlablenkeinheit (4) ein zweiachsiges Spiegelsystem (12) zum Steuern des materialbearbeitenden Laserstrahls (3) entlang der zu bearbeitenden Oberfläche der Probe (1) gemäß des Abtragmusters aufweist.

10. Vorrichtung nach Anspruch 6,
**dadurch gekennzeichnet, dass** die Probeneinspanneinheit (5) mit einer CNC-Kinematik ausgestattet ist, um die zu bearbeitende Probe (1) räumlich gegenüber der relativ ortsfest hierzu angeordneten Strahlablenkeinheit (4) zu positionieren.

## Claims

1. Method for preparing a micrograph of a metallurgical sample (1), with the preparation of the sample (1) being followed by thermal contrasting of the surface by laser means,
**characterized in that**
at least the following processing steps are carried out by a USP laser (2):
- cleaning (R) the surface of the sample (1) with a removal depth between 1 and 50 µm in order to expose joint gap structures (300),
- contrasting (K) the cleaned surface of the sample (1) with a removal depth between 0.5 and 20 µm in order to render microstructures (400) visible,
- laser etching (A) the contrasted surface of the sample (1) by producing, without material removal, a thermally induced temper-colour structure (500) and/or surface oxidation.

2. Method according to Claim 1,
**characterized in that**
cleaning (R) is preceded by smoothing (G) the surface of the sample (1) with a removal depth between 20 and 150 µm in order to level out rough surfaces.

3. Method according to Claim 1,
**characterized in that**
the USP laser (2) for laser etching (A) is operated at a constant wavelength 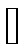 in the range between 1020 nm and 1040 nm, preferably 1030 nm +/- 3 nm, and with a pulse duration (t_{P}) of ≤10 ps.

4. Method according to Claim 1 or 2,
**characterized in that**
at least the material-removing steps of cleaning (R) and contrasting (K) are carried out in accordance with a uniform linear removal pattern.

5. Method according to Claim 4,
**characterized in that**
the removal pattern is applied with at least 6 hatch directions, preferably 8 hatch directions, at an angle of 40° to 50°, preferably 45°, per removal pass.

6. Device for carrying out the multi-step method according to any of the preceding claims, at least comprising a USP laser (2) for generating a laser beam (3), a beam deflection unit (4) for moving and focusing the laser beam (3) relative to the surface of the sample (1), a sample clamping unit (5) for positioning the sample (1) relative to the beam deflection unit (4), and an electronic control unit (6) for coordinated control of these units in accordance with the method step to be carried out (G; R; K; A).

7. Device according to Claim 6,
**characterized in that** a focus shifter (10) for positioning the laser focus along the optical Z axis is arranged between the USP laser (2) and the beam deflection unit (4).

8. Device according to Claim 7,
**characterized in that** a beam expansion unit (11) for increasing the laser beam cross section is arranged between the USP laser (2) and the focus shifter (10).

9. Device according to Claim 6,
**characterized in that** the beam deflection unit (4) comprises a two-axis mirror system (12) for controlling the material-processing laser beam (3) along the surface of the sample (1) to be processed in accordance with the removal pattern.

10. Device according to Claim 6,
**characterized in that** the sample clamping unit (5) is equipped with CNC kinematics in order that the sample (1) to be processed is positioned spatially opposite the beam deflection unit (4) arranged relatively fixed in position with respect thereto.

## Revendications

1. Procédé de préparation d'un motif de broyage d'un échantillon métallurgique (1), dans lequel un contraste thermique de la surface est réalisé par un équipement laser après préparation de l'échantillon (1),
**caractérisé en ce que**
au moins les étapes de traitement suivantes sont réalisées à l'aide d'un laser UKP (2) :
- nettoyage (R) de la surface de l'échantillon (1) avec une profondeur d'ablation comprise entre 1 et 50 µm pour révéler des structures d'interstice de jonction (300),
- contraste (K) de la surface nettoyée de l'échantillon (1) avec une profondeur d'ablation comprise entre 0,5 et 20 µm pour rendre visibles des structures de jonction (400),
- gravure au laser (A) de la surface contrastée de l'échantillon (1) par le biais de la création sans ablation d'une structure de couleur de recuit thermique (500) et/ou oxydation de surface.

2. Procédé selon la revendication 1,
**caractérisé en ce que**
avant le nettoyage (R), un lissage (G) de la surface de l'échantillon (1) avec une profondeur d'ablation comprise entre 20 et 150 µm est effectué pour aplanir les surfaces rugueuses.

3. Procédé selon la revendication 1,
**caractérisé en ce que**
le laser UKP (2) est mis en œuvre pour la gravure au laser (A) avec une longueur d'onde constante 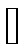 dans la plage comprise entre 1020 nm et 1040 nm, de préférence 1030 nm +/- 3 nm, ainsi qu'avec une durée d'impulsion (t_{P}) ≤ 10 ps.

4. Procédé selon la revendication 1 ou 2,
**caractérisé en ce que**
au moins les étapes d'ablation de matière comprenant le nettoyage (R) et le contraste (K) sont effectuées selon un modèle d'ablation linéaire uniforme.

5. Procédé selon la revendication 4,
**caractérisé en ce que**
le motif d'ablation est appliqué avec au moins 6 directions de hachurage, de préférence 8 directions de hachurage, selon un angle de 40° à 50°, de préférence 45°, par ablation.

6. Dispositif pour la mise en œuvre du procédé en plusieurs étapes selon l'une des revendications précédentes, comprenant au moins un laser UKP (2) pour la génération d'un faisceau laser (3), une unité de déviation de faisceau (4) pour le déplacement et la focalisation du faisceau laser (3) par rapport à la surface de l'échantillon (1), une unité de serrage d'échantillon (5) pour le positionnement de l'échantillon (1) par rapport à l'unité de déviation de faisceau (4), ainsi qu'une unité de commande électronique (6) pour le pilotage coordonné de ces unités en fonction de l'étape de procédé à mettre en œuvre (G ; R ; K ; A).

7. Dispositif selon la revendication 6,
**Caractérisé en ce qu'**entre le laser UKP (2) et l'unité de déviation de faisceau (4) est disposé un dispositif de décalage de foyer (10) pour le positionnement du foyer laser le long de l'axe optique Z.

8. Dispositif selon la revendication 7,
caractérisé en ce sens qu'entre le laser UKP (2) et le dispositif de décalage de foyer (10) est disposée une unité d'élargissement de faisceau (11) pour agrandir la section transversale du faisceau laser.

9. Dispositif selon la revendication 6,
**caractérisé en ce que** l'unité de déviation de faisceau (4) comporte un système de miroirs à deux axes (12) pour commander le faisceau laser de traitement de matière (3) le long de la surface à traiter de l'échantillon (1) conformément au motif d'ablation.

10. Dispositif selon la revendication 6,
**caractérisé en ce que** l'unité de serrage d'échantillon (5) est équipée d'une cinématique CNC pour positionner l'échantillon à traiter (1) dans l'espace par rapport à l'unité de déviation de faisceau (4) disposée de manière relativement fixe par rapport à celui-ci.
